# EUROPÄISCHE PATENTSCHRIFT

(11) **EP 0 924 522 B1**
(45) Veröffentlichungstag und Bekanntmachung des Hinweises auf die Patenterteilung: **16.08.2006**
(21) Anmeldenummer: 98122039.5
(22) Anmeldetag: 20.11.1998
(51) Int. Cl.: G01N 33/542, G01N 33/569, G01N 33/68, G01N 33/92, G01N 33/58, G01N 33/543

(54) **Nachweis und Bestimmung festphasenassoziierter Faktoren**
Detection and determination of solid phase associated factors
Détection et détermination des facteurs associés à une phase solide

(30) Priorität: 19.12.1997 DE 19756782
(43) Veröffentlichungstag der Anmeldung: 23.06.1999
(73) Patentinhaber: Dade Behring Marburg GmbH, 35001 Marburg (DE)
(72) Erfinder: Kraus, Michael Dr., 35041 Marburg (DE); Schelp, Carsten Dr., 35041 Marburg (DE); Schuy, Wilhelm Dr., 56414 Obererbach (DE)

(56) Entgegenhaltungen:
- EP-A- 0 515 194
- GUO C. ET AL: "FLUORESCENCE RESONANCE ENERGY TRANSFER REVEALS INTERLEUKIN (IL)-1-DEPENDENT AGGREGATION OF IL-1 TYPE I RECEPTORS THAT CORRELATES WITH RECEPTOR ACTIVATION" J. BIOL. CHEM., Bd. 270, Nr. 46, 1995, Seiten 27562-27568, XP000882822
- KOKSCH M. ET AL.: "FLUORESCENCE RESONANCE ENERGY TRANSFER AS A NEW METHOD FOR THE EPITOPE-SPECIFIC CHARACTERIZATION OF ANTI-PLATELET ANTIBODIES" J. IMMUN. METH., Bd. 187, 1995, Seiten 53-67, XP002134855
- KUBITSCHECK U. ET AL.: "FLUORESCENCE RESONANCE ENERGY TRANSFER ON SINGLE LIVING CELLS" BIOPHYS. J., Bd. 60, 1991, Seiten 307-318, XP000882076
- DAMJANOVICH S. ET AL: "PREASSEMBLY OF INTERLEUKIN 2 (IL-2) RECEPTOR SUBUNITS ON RESTING KIT 225 K6 T CELLS AND THEIR MODULATION BY IL-2, IL-7 AND IL-15: A FLUORESCENCE RESONANCE ENERGY TRANSFER STUDY" PNAS, Bd. 94, November 1997 (1997-11), Seiten 13134-13139, XP000882829
- SZÖLLÖSI J. ET AL: "APPLICATION OF FLUORESCENCE RESONANCE ENERGY TRANSFER IN THE CLINICAL LABORATORY: ROUTINE AND RESEARCH" CYTOMETRY, Bd. 34, Nr. 4, August 1998 (1998-08), Seiten 159-179, XP000882220
- YEGNESWARAN S. ET AL.: "PROTEIN S ALTERS THE ACTIVE SITE LOCATION OF ACTIVATED PROTEIN C ABOVE THE MEMBRANE SURFACE" J. BIOL. CHEM., Bd. 272, Nr. 40, Oktober 1997 (1997-10), Seiten 25013-25021, XP000882837

## Beschreibung

Die vorliegende Erfindung betrifft Verfahren zum Nachweis oder zur Bestimmung festphasenassoziierter Faktoren, welche mit derselben Festphase mehrfach assoziiert sind. Erfindungsgemäß wird die Probe mit einem Senderpartikel, an dem mindestens ein Ligand mit Bindungsaffinität für einen festphasenassoziierten Faktor und ein Sender immobilisiert sind, und einem Empfängerpartikel, an dem mindestens ein Ligand mit Bindungsaffinität für besagten festphasenassoziierten Faktor und ein Empfänger immobilisiert ist, in Kontakt gebracht, und anschließend wird das Signal bestimmt, welches dann entsteht, wenn Sender und Empfänger in ausreichende Nähe zueinander gebracht werden. Insbesondere betrifft die Erfindung den Nachweis von zellulären Oberflächenrezeptoren, die zur Typisierung von Zellen oder zur Bestimmung zellulärer Aktivierungszustände verwendet werden können. Damit ist es möglich, die bisher vielfach übliche Durchflußzytometrie durch ein einfacheres Verfahren zu ersetzen.

Die Differenzierung von Blutzellen, insbesondere der Leukozyten (Granulozyten, Monozyten und Lymphozyten) ist ein routinemäßig angewandtes und wichtiges Verfahren in der Diagnostik. Sie basiert u.a. darauf, daß verschiedene Zelltypen durch verschiedene Oberflächenantigene charakterisiert sind, wie beispielsweise Membranproteine aus der Familie der Integrine (Hynes RO. Integrins: a family of cell surface receptors. Cell 1987; 48: 549-554). Die meisten dieser Membranproteine sind mit CD-Nummern ("cluster designation numbers") bezeichnet.

Membranproteine können auch erst nach Stimulierung der Zellen auf der Oberfläche exponiert oder durch Verschmelzung intrazellulärer Vesikel mit der Oberfläche sezerniert werden, wie beispielsweise Proteine aus der Gruppe der Selektine (Bevilacqua MP and Nelson RM. Endothelial-Leukocyte adhesion molecules in inflammation and metastasis. Thromb. Haemost. 1993; 70: 152-154).

Bei Plättchen ist beispielsweise GMP-140 (P-Selectin; DC62P) ein Marker einer Aktivierung. Weiterhin können bei Aktivierungszuständen charakteristische Komplexe von Rezeptoren von Zellen und Liganden entstehen, wie beispielsweise auf aktivierten Plättchen Komplexe der Glykoproteine GP Ib/IX oder GP IIb/ IIIa, die von Willebrand-Faktor bzw. Fibrinogen binden (siehe beispielsweise Clemetson KJ. Biochemistry of platelet membrane glycoproteins, Prog. Clin. Biol. Res. 1988; 283:33-75). Auf Plättchen werden nach Aktivierung auch phosphatidylserinhaltige Lipidmembranen exponiert, an die sich Gerinnungsfaktoren oder andere phospholipidbindende Protein (beispielsweise aus der Familie der Annexine) binden können.

In bisherigen Verfahren werden markierte Antikörper oder andere markierte affine Liganden, beispielsweise Annexine für den Nachweis von phosphatidylserinhaltigen Lipidmembranen (Römisch J et al., Anticoagulant properties of placenta protein 4 (annexin V); Thromb. Res. 1990; 60: 355-366), gegen diese Oberflächenantigene zu Blut gegeben. Mittels Durchflußzytometrie werden dann die Zellen nach ihrer Größe sortiert und dabei gleichzeitig über den Nachweis der Markierung auf die Anzahl und Anteile eines oder parallel mehrerer Zelltypen geschlossen. Als Markierungen dienen dem Fachmann an sich bekannte Substanzen, insbesondere chemilumineszente Verbindungen (für einen Überblick siehe beispielsweise Michelson, A.D. und Barnard, M.R., US 5 552 290).

Die obengenannte Durchflußzytometrie ist ein etabliertes Verfahren, hat aber den Nachteil, daß sie nur für den speziellen Zweck der Zellzählung und/ oder - typisierung verwendet werden kann. Meist ist sie daher auch nur in speziellen Labors etabliert. Eine breitere Anwendung der Differenzierung von Zellen und deren Aktivierungszuständen wäre für klinische Fragestellungen aber wünschenswert. Für die Routineanwendung ist die Applikation an gängigen klinisch-chemischen Analysern oder anderen Automaten für das Routinelabor notwendig.

Der Erfindung lag daher die Aufgabe zugrunde, eine Alternative zu den bisher üblichen durchflußzytometrischen Methoden bereitzustellen, die die Bestimmung von Zelloberflächenantigenen in einem homogenen, immunchemischen Verfahren erlaubt.

Eine Reihe von homogenen, immunchemischen Verfahren zur Bestimmung von Antigenen bzw. Antikörpern sind bereits bekannt, wie beispielsweise das FRAT®⁻System (Fa. Syva), das EMIT® -System, die Enzym Channeling Immunoassays, die Fluoreszenz Energie Transfer Immunoassays (FETI, z. B. TRACE® Technology; Fa. CIS bio International), die Enzym Inhibitor Immunoassays (Hoffmann LaRoche, Abbott Laboratories) die Fluorescence Polarization Immunoassays (Dandlicker), Photoaktive Chemilumineszenzmatrices (WO94/03812) oder homogene Techniken auf Basis von Kryptaten seltener Erden (Clin. Chem., Bd. 41, Nr. 9, 1995, 1391-1397). Diese homogenen Verfahren wurden entwickelt, um Methoden anzubieten, die ohne Trenn- und/ oder Waschschritte durchgeführt werden können. Einige dieser Verfahren besitzen nur eine begrenzte Sensitivität oder eignen sich nicht zur Bestimmung von höhermolekularen Analyten mit multiplen Epitopen.

Der Ausdruck Scintillation proximity assay (SPA) wurde von Hiram E. Hart und Elaine B. Greenwald eingeführt (Molecular Immunology 1979; 16: 265 - 267), um einen bestimmten homogenen Radioimmunoassay zu beschreiben. Bei diesem Verfahren werden zwei verschiedene Arten von polymeren Beads eingesetzt, die mit spezifischen Bindungspartnern beladen sind. Die erste dieser Bead-Arten ist zusätzlich mit einem Farbstoff beladen während die zweite Bead-Art zusätzlich Tritium trägt. Der Farbstoff hat die Eigenschaft, Lichtimpulse zu emittieren, sobald er von der ³H β-Strahlung (Auger-Elektronen) angeregt wird. Diese Strahlung hat aber nur eine Reichweite von wenigen Micrometern in wässrigen Lösungen, so daß in verdünnten Suspensionen, die beide Bead-Arten enthalten, sich nur wenige Beads der einen Art in ausreichender Nähe zu Beads der anderen Art befinden. Dadurch kann insgesamt nur ein geringes Fluoreszenzsignal entstehen. Durch die Zugabe von Reaktanden, die mit den spezifischen Bindungspartnern der beiden Bead-Arten reagieren können, findet aber eine Aggregation der Beads statt, die viele der Beads der ersten Art (Tritium-Beads) in die Nähe von Beads der zweiten Art (Fluorophor-Beads) bringen, so daß ein insgesamt höheres Signal entsteht. Das entstehende Signal wird in einem Scintillationszähler erfaßt. Eine Weiterentwicklung dieses Verfahren durch Verwendung von ¹²⁵Iod markierten spezifischen Bindungspartnern wurde von Udenfriend, S. et al. beschrieben (Proc. Natl. Acad. Sci. 1985; 82: 8672 - 8676).

Ein weiteres Verfahren wird als "luminescent oxygen channeling immunoassay (LOCI)" bezeichnet (EP-0 515 194 A2; Ullman et al., Proc. Natl. Acad. Sci. 1994; 91: 5426 - 5430; Ullman et al., Clinical Chemistry 1996; 42: 1518 - 1526). Darin werden zwei Partikeltypen verwendet, von denen einer einen Photosensitizer (Sensitizer-Beads) und der andere eine chemilumineszente Komponente (Acceptor-Beads) enthält. Der Photosensitizer generiert Singulettsauerstoff und aktiviert die chemilumineszente Komponente, wenn sie in ausreichender Nähe ist. Die aktivierte chemilumineszente Komponente generiert Licht, welches als Meßsignal erfaßt werden kann.

Bystrak, S. et al. (Analytical Biochemistry 1995; 225: 127 - 134) beschreiben ein homogenes Verfahren, bei dem eine Photooxidation durch Singulettsauerstoff eines Fluoreszenzsubstrates stattfindet, welches in einem unilaminaren Vesikel eingebunden ist. Auf der Oberfläche des Vesikels sind spezifische Bindungspartner kovalent gebunden.

Alle diese Verfahren sind durch eine spezifische Bindung von Partikeln an Bindungspartner gekennzeichnet. Die Bindung an diese Bindungspartner erfolgt in der Regel über die Beschichtung der Partikel mit entsprechenden spezifischen Liganden, wie etwa Antigenen oder Antikörpern zum immunchemischen Nachweis. Bisher wurden diese Verfahren nur zum Nachweis von löslichen (humoralen) Faktoren angewendet. Bei Bindung an diese Faktoren (beispielsweise Proteine) werden Sender- und Empfängerpartikel in eine räumliche Nähe gebracht, die eine Übertragung der von einem Sender abgegebenen Energie auf ein Empfängerpartikel erlaubt. Eine Anwendung zum Nachweis von unlöslichen, festphasenassoziierten Faktoren, wie beispielsweise Zelloberflächenantigenen, wurde bisher nicht gezeigt.

### Zusammenfassung der Erfindung

Überraschenderweise wurde im Rahmen vorliegender Erfindung festgestellt, daß Sender- und Empfängerpartikel so an einen festphasenssoziierten Faktor, welcher mehrfach festphasenassoziert ist, gebunden werden können, daß die zur Energieübertragung erforderliche räumliche Nähe unabhängig von der Größe der Festphase erreicht wird oder, mit anderen Worten, daß die größte Entfernung zwischen Senderpartikel und Empfängerpartikel, bei der Energieübertragung noch stattfinden kann, nicht überschritten wird. Bei der Festphase handelt es sich um eine Zelle und bei dem festphaseassoziierten Faktor beispielsweise um ein zelluläres Oberflächenantigen handeln. Es konnte somit überraschenderweise gezeigt werden, daß die Ausdehnung homogener immunchemischer Nachweisverfahren, die bisher ausschließlich auf den Nachweis humoraler Faktoren beschränkt waren, auf den Nachweis von Zelloberflächenmarkern möglich ist, so daß deren Bestimmung auch auf anderen, als auf dem Prinzip der Durchflußzytometrie beruhenden Geräten erfolgen kann.

Die vorliegende Erfindung betrifft ein homogenes immünchemisches Verfahren welches nicht auf dem Prinzip der Durchflusszytometrie beruht und welches auf anderen als auf dem Prinzip der Durchflusszytometrie beruhenden Geräten ausgewertet wird, zum gleichzeitigen Nachweis oder zur gleichzeitigen Bestimmung voneinander verschiedener festphasenassoziierter Faktoren, nämlich mindestens eines ersten festphasenassoziierten Faktors Fx und eines zweiten festphasenassoziierten Faktors Fy, wobei Fx und Fy mit derselben Festphase assoziiert sind, in einer Probe. Erfindungsgemäß wird die Probe mit mindestens mit einem ersten stabilen Komplex, bestehend aus mindestens einem Liganden Lx, der Bindungsaffinität für Fx besitzt und einem Sender S, wobei Lx zusammen mit S an einem ersten Partikel immobilisiert ist, sowie einem zweiten stabilen Komplex, bestehend aus mindestens einem Liganden Ly, der Bindungsaffinität für Fy besitzt und einem Empfänger E, wobei Ly zusammen mit E an einem zweiten Partikel immobilisiert ist, in Kontakt gebracht, so daß sich Komplexe Fx-Lx-S und Fy-Ly-E ausbilden. Es wird das Signal bestimmt, welches dann entsteht, wenn sich S und E in ausreichender Nähe zueinander befinden und worin die Festphase eine in der Flüssigkeit suspendierte Zelle und Fx und/oder Fy ein integrales Membranprotein, ein membranassozüertes Protein oder ein Lipid ist.

Die Festphase ist eine Zelle, beispielsweise ein Erythrozyt, Leukozyt, Granulozyt, Lymphozyt, Monozyt, Thrombozyt, oder eine Zelle aus einem anderen Gewebe oder Organ. Der Begriff Zelle kann erfindungsgemäß aber auch eine prokaryontische oder eukaryontische körperfremde Zelle, wie ein Bakterium oder Parasit, oder auch einen subzellulären Parasiten, beispielsweise ein Virus bedeuten.

Unter festphasenassoziierten Faktoren sind sowohl solche Faktore zu verstehen, die in die Festphase integriert sind, als auch solche Faktoren, die nicht in die Festphase integriert, sondern aufgrund anderer Wechselwirkungen mit ihr assoziiert sind.

Als Probenmaterial kommen beispielsweise Körperflüssigkeit, Gewebeextrakt oder ex vivo-Kulturen in Frage. Dabei handelt es sich bei Körperflüssigkeiten vorzugsweise um Blut, Synovialflüssigkeit, Cerebrospinalflüssigkeit, Aszites oder Urin, besonders bevorzugt um Vollblut oder Plättchen-reiches Plasma.

Vorliegende Erfindung betrifft weiterhin ein Verfahren, worin F, Fx und/oder Fy ein integrales Membranprotein, ein membranassoziiertes Protein, eine Glycostruktur oder ein Lipid ist. Das integrale Membrabprotein kann dabei beispielsweise ein Integrin, Selektin, ein Protein aus dem MHC-Komplex oder ein anderes bekanntes Protein nach der "cluster designation" sein. Membranassoziierte Proteine sind nicht in der Membran integriert, sondern über spezifische Liganden/Rezeptor-Wechselwirkungen auf der Oberfläche nachweisbar, wie beispielsweise Fibrinogen an Fibrinogen-Rezeptoren, Antikörper gegen Membranproteine, Komplementfaktoren oder Lectine gegen Kohlenhydratstrukturen auf der Membranoberfläche und/oder -proteinen oder prozessiertes Antigen im MHC-Komplex auf Antigen-präsentierenden Zellen. Es handelt sich bei den membranassoziierten Proteinen weiterhin um Proteine, die über elektrostatische Wechselwirkungen auf der Oberfläche nachweisbar sind, wie beispielsweise aktive Enzyme des Gerinnungssystems oder Proteine aus der Familie der Annexine. Bei den Lipiden handelt es sich erfindungsgemäß um dem Fachmann bekannte Substanzen aus der Gruppe der Acylglycerine, Phosphoglyceride, Sphingolipide, Wachse, Terpene, Steroide und/oder Prostaglandine. Erfindungsgemäß bevorzugterweise wird die Zusammensetzung der Phospholipide der Oberflächenmembran, wie beispielsweise der Anteil an Phosphatidylserin bzw. an Phosphatidylethanolamin durch Bindung affiner Liganden, wie Proteine aus der Familie der Annexine bzw. durch Bindung affiner Proteine des Gerinnungssystems wie etwa aktiviertes Protein C oder Protein S nachgewiesen.

Die vorliegende Erfindung betrifft außerdem Verfahren, worin der Ligand über einen vermittelnden Bindungspartner an F, Fx oder Fy bindet.

Weiterhin betrifft die vorliegende Erfindung Verfahren, worin L, Lx oder Ly über eine Biotin-Avidin-Brücke an Partikel gebunden wird.

Vorliegende Erfindung betrifft daüberhinaus Verfahren, worin L, Lx oder Ly ein Antikörper, Antigen, Lektin, Coenzym, Apoprotein, Ligand eines Rezeptors, Substratanaloges oder Annexin sein kann.

Gemäß einer bevorzugten Ausführungsform vorliegender Erfindung findet zwischen dem Sender S und dem Empfänger E ein Energietransfer statt. Dieser kann beispielsweise durch radioaktive Prozesse, durch Anregung photosensibler Farbstoffe und dadurch bedingter direkter oder indirekter Elektronenübertragung, beispielsweise mittels aktiviertem Sauerstoffs, erfolgen. Gemäß einer weiteren Ausführungsform vorliegender Erfindung führt der Energietransfer beim Empfänger-Partikel zu einer Reaktion, beispielsweise einer Emission von Lumineszenz, bevorzugterweise Chemilumineszenz, oder Fluoreszenz, die nachweisbar und ein Maß für die räumliche Nähe von Sender- und Empfänger-Partikel ist.

Erfindungsgemäß können auch durch den Zusatz von den dem Fachmann im jeweiligen System bekannten, den Energietransfer modulierenden, beispielsweise dämpfenden Substanzen, wie zum Beispiel Farbstoffen oder Antioxidantien, der notwendige Mindestabstand zwischen Sender- und Empfängerpartikel verringert und damit Meß- / Hintergrund-Signal Verhältnis verbessert werden.

Das erfindungsgemäße Verfahren kann beispielsweise der Charaktierisierung von Zelltypen, Untergruppen oder Aktivierungszuständen von Zellen dienen, sowie dem Nachweis von Oberflächenmarkern oder Oberflächenantigenen, beispielsweise Neoepitopen im Rahmen einer Tumorentstehung auf Zellen. Es kann auch der Typisierung von Geweben oder der Charakterisierung der Gewebekompatibilität dienen. Insbesondere ist auch die Identifikation von körperfremden Zellen, allgemein Krankheitserregern, wie Bakterien Gegenstand der vorliegenden Erfindung. Ganz besonders eignet sich das erfindungsgemäße Verfahren zur Identifikation von Chlamydien.

Unter "Sender" und "Empfänger" sind im Rahmen der vorliegender Erfindung Mitglieder biologischer oder chemischer Stoffklassen zu verstehen, die bei räumlicher Nähe miteinander in Wechselwirkung treten können, z.B. in Form von Energiespendern und Energieempfängern, wie beispielsweise Photosensitizer und Chemiluminescer (EP-0 515 194; Ullman et al. (1996) Clinical Chemistry 42:1518 - 1526), Photosensitizer und Fluorophore (WO 95/06877; Bystrak et al. (1995) Anal. Biochem. 225:127 - 134), oder radioaktives Iod¹²⁵ und Fluorophore (S. Udenfriend et al. (1985) Proc. Natl. Acad. Sci. 82:8672 - 8676), oder Fluorophore und Fluorophore (Mathis,G. (1993) Clin. Chem. 39:1953 - 1959) oder Fluorophore und Fluoreszenz-Quencher (US 3,996,345). Der Energietransfer kann hierbei von einer auf eine andere Substanz erfolgen, möglich ist aber auch eine Kaskade verschiedener Substanzen über die der Energietransfer läuft.

Unter einer Wechselwirkung zwischen Sender und Empfänger ist insbesondere ein Energietransfer - also die direkte Übertragung von Energie zwischen Sender und Empfänger, beispielsweise durch Licht- oder Elektronenstrahlung sowie über reaktive chemische Moleküle, zu verstehen.

Desweiteren sind unter dem Begriff einer Wechselwirkung zwischen Sender und Empfänger auch Enzymkaskaden zu verstehen. In diesem Fall sind die Substanzen Enzyme, von denen mindestens eines das Substrat für ein anderes liefert.

Ferner umfaßt sind hiervon auch Vorgänge, bei denen die Aktivität einer Substanz durch eine oder mehrere andere inhibiert oder verstärkt wird, beispielsweise die Hemmung oder Steigerung der Enzymaktivität oder die Hemmung, Steigerung oder Veränderung (z.B. Wellenlängenverschiebung) des von der beeinflußten Substanz ausgesendeten Lichtes.

Eine effektive Wechselwirkung zwischen Sender und Empfänger findet statt, wenn diese räumlich benachbart vorliegen, also z.B. innerhalb eines Abstandbereiches von wenigen µm, insbesondere innerhalb eines Abstandbereiches von unter 600 nm, bevorzugt unter 400nm, ganz besonders bevorzugt von unter 200 nm.

In einer bevorzugten Ausführungsform des erfindungsgemäßen Verfahrens erfolgt die Wechselwirkung zwischen Sender und Empfänger als Energietransfer z.B. mittels
- kurzlebiger Moleküle, z.B. Singulett-Sauerstoff (s.a. EP 0 515 194; Ullman et al. (1994) Proc. Natl. Acad. Sci. 91:5426-5430; Ullman et al. (1996) Clinical Chemistry 42:1518 - 1526, WO 95/06877 und Bystrak et al. (1995) Anal. Biochem. 225: 127 - 134),
- Strahlung geringer Reichweite, z.B. radioaktive β-Strahlung (s. Hart & Greenwald (1979) Molecular Immunology 16:265-267 und Udenfriend et al. (1985) Proc. Natl. Acad. Sci. 82:8672-8676),
und/oder Energietransfers nach Förster (Mathis, G. (1993) Clin. Chem. 39:1953-1959; US 5,527,684).

Vom erfindungsgemäßen Verfahren eingeschlossen sind auch Ausführungsformen bei denen die Oberfläche der Partikel nach deren Herstellung weiter modifiziert wurde und/oder die Partikel von einer oder mehreren kovalent oder adsorbtiv gebundenen Schichten oder Schalen, beispielsweise aus Proteinen, Kohlehydraten, lipophilen Substanzen, Biopolymeren, organischen Polymeren oder Mischungen hiervon umhüllt sind, um beispielsweise Verbesserungen zu erreichen hinsichtlich Suspensionsstabilität, Lagerstabilität, formgebende Stabilität oder Resistenz gegen UV-Licht, Mikroben oder sonstige zerstörend wirkende Agenzien. Ebenfalls können die Modifikationen u. Umhüllungen dazu dienen die unspezifische Bindung an Oberflächen von Reaktionsgefäßen sowie an die von Probenbestandteilen wie insbesondere Proteinen (z.B. Albumin oder Antikörper) oder Zellbestandteilen (zum Beispiel Phospholipiden oder Nukleinsäuren) zu reduzieren oder zu unterbinden. Weiterhin können die Modifikationen u. Umhüllungen dazu dienen die Hydrophobizität der Partikeloberfläche oder die Ladung der Oberfläche der Partikel zu erhöhen oder zu erniedrigen.

Eine weitere Ausfühnmgsform des erfindungsgemäßen Verfahrens ist dadurch gekennzeichnet, daß als Sender bzw. Empfänger Photosensitizer, beispielsweise Aceton, Benzophenon, 9-Thioxanthone, Eosin, 9,10-Dibromoanthrazen, Chlorophyll, Buckminsterfüllerene, Methylen Blau, Rose Bengal, Porphyrine, Phthalocyanine und/oder deren Derivate, und als chemilumineszierende Verbindungen, beispielsweise Olefine, 9-Alkylidenexanthane, 9-Alkylidene-N-alkylakridane, Enolether, Enamine, Arylvinylether, Dioxene, Arylimidazole und/oder Lucigenin eingesetzt werden und der vom Photosensitizer generierte Singulett-Sauerstoff die chemilumineszierenden Verbindungen zur Lichtaussendung aktivieren kann. Bevorzugt wird im erfindungsgemäßen Verfahren auch die Verwendung von Stoffen wie z. B. Luminol und Oxalatester, die mit Singulett-Sauerstoff zu Intermediaten reagieren, welche mit dem Fachmann bekannten Reagenzien unter Lichtaustrahlung reagieren können.

Die chemilumineszierenden Verbindungen emmittieren in der Regel Licht in den Wellenlängenbereichen über 300 nm. Die Fluoreszenz von Plasma sinkt rapide im Bereich von 500 nm und kann über 550 nm vernachlässigt werden. Werden höhere Wellenlängen gefordert, können die chemilumineszierenden Verbindungen erfindungsgemäß auch mit Fluorophoren in Kontakt gebracht werden, die von den aktivierten chemilumineszierenden Verbindungen angeregt werden können und bei höheren Wellenlängen emittieren. Geeignete Fluorophore sind z.B. Rhodamine, Ethidiumbromid, 5-Dimethylaminonapthalen-1-sulfonyl, Europiumchelate mit dem Agenz 3-(2-thienoyl)-1,1,1-trifluoroaceton [Eu(TTA)₃ (TTA = 3-(2-thienoyl)-1,1,1-trifluoroaceton)] oder Rutheniumchelate mit dem Agenz 2,2'-dipyridyl [Ru(bpy)₃⁺⁺ (bpy = 2,2'-dipyridyl)].

Eine weitere Ausführungsform des erfindungsgemäßen Verfahrens ist dadurch gekennzeichnet, daß als Substanzen Photosensitizer und fluoreszierende Verbindungen eingesetzt werden und der vom Photosensitizer generierte Singulett-Sauerstoff die fluoreszierende Verbindung zur Lichtaussendung aktivieren bzw. in einem Quenchprozess die Lichtaussendung unterdrücken kann. Insbesondere werden erfindungsgemäße Verfahren bevorzugt, die durch den Einsatz fluoreszierender Verbindungen gekennzeichnet sind, die durch Reaktion mit Singulett-Sauerstoff einer Photooxidation - einem "Photobleaching" - unterliegen, wie zum Beispiel 1,3-di-Phenylisobenzofuran, oder mit Singulett-Sauerstoff als photoaktive Vorstufen zu Fluorophoren reagieren, wie zum Beispiel Oxene umbelliferylether oder Umbelliferylselenide.

Hinsichtlich weiterer Beispiele für im Hinblick auf das erfindungsgemäße Verfahren geeignete Partikel, Photosensitizer, chemilumineszierende oder fluoreszierende Verbindung wird insbesondere auf EP 0 515 194, Ullman et al. (Proc. Natl. Acad. Sci. 91:5426-5430, 1994) u. Ullman et al. (Clinical Chemistry 42:1518-1526, 1996, WO 95/06877) verwiesen.

### Detaillierte Beschreibung der Erfindung

Da eine Festphase im Sinne vorliegender Erfindung, beispielsweise eine Zelle, als polyvalent hinsichtlich eines festphasenassoziierten Faktors, beispielsweise eines Oberflächenepitops betrachtet werden kann, müssen nicht notwendigerweise zwei verschiedene Bindungspartner auf Sender- und Empfängerpartikel aufgebracht werden. Es genügt somit der gleiche Bindungspartner auf Sender- und Empfängerpartikeln zum Nachweis bestimmter festphasengebundener Faktoren, beispielsweise von Antigenen oder Liganden. Damit wird die Bestimmung bestimmter Zelltypen oder die Bestimmung bestimmter Aktivierungszustände ermöglicht, die mit der Ausprägung bestimmter Faktoren auf der Zelloberfläche einhergehen.

Darüberhinaus können aber auch zwei oder mehr verschiedene Bindungspartner auf Sender- und Empfängerpartikel aufgebracht werden, um zusätzlich zur Detektion einzelner Faktoren, wie beispielsweise einzelner Antigene, weitere Differenzierungen zu erlauben. Dazu muß aber der Radius der effektiven Energieübertragung so niedrig sein, daß sie wirklich nur zwischen sehr eng benachbarten Sender- und Empfängerpartikeln erfolgen kann und die Polyvalenz der Festphase selbst - beispielsweise aufgrund der starken Häufung eines der Bindungspartner, zu keiner unerwünschten Energieübertragung führt. Der Radius der effektiven Energieübertragung ist abhängig vom verwendeten Nachweissystem, und die Reduktion dieses Radius kann durch die Veränderung der Beschaffenheit der Partikel oder die Beeinflussung des Quencheffekts der umgebenden Lösung mittels dem Fachmann bekannter Zusätze erreicht werden.

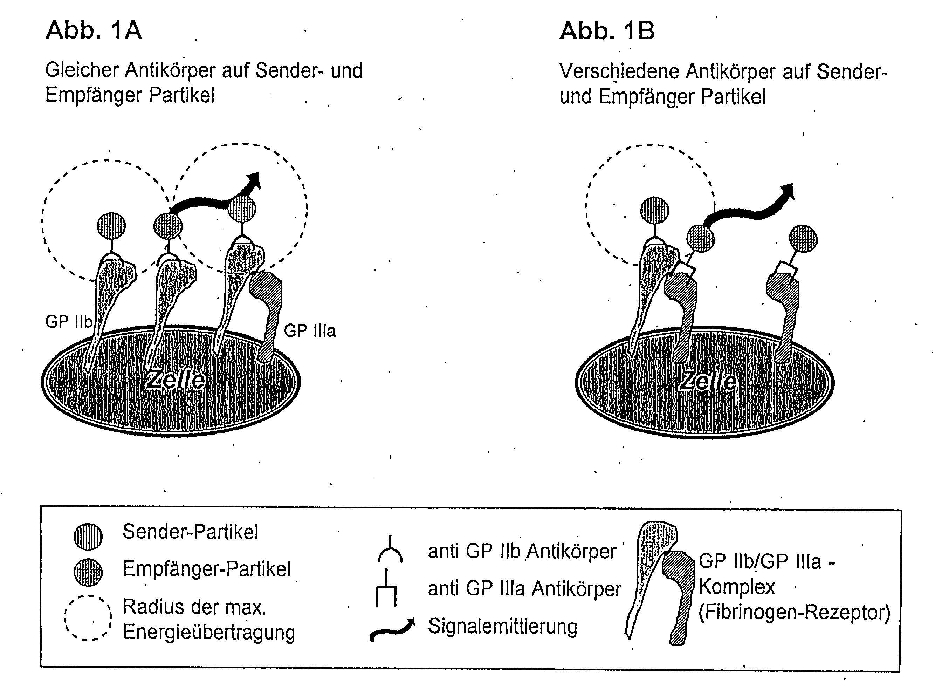

Zur näheren, beispielhaften Erläuterung wird auf Abb. 1 verwiesen. Gemäß Abb. 1A wurden Sender- und Empfängerpartikel mit dem gleichen Antikörper gegen GP IIb beladen. Eine Übertragung der Energie vom Sender auf den Empfänger und damit eine Signalemission tritt nur auf, wenn die GP IIb Moleküle so dicht auf der Zelloberfläche angeordnet sind, daß die mittlere Entfernung der Moleküle voneinander kleiner ist als der maximale Radius, innerhalb dessen Energieübertragung zwischen Sender- und Empfängerpartikel noch möglich ist. Die Signalübertragung ist unter diesen Umständen ein Maß für die Quantitität (die Häufgkeit auf der Oberfläche) eines bestimmten Oberflächenepitops, im vorliegenden Beispiel des GP IIb.

Von Interesse ist beispielsweise die Bestimmung einzelner Oberflächenantigene, wie T4 und T8 zur Differenzierung von Lymphozyten, IgE-Rezeptoren zum Nachweis allergener Reaktionen, oder die Bestimmung der Histokompatibilitätsantigene in Zellextrakten oder -lysaten vor Transplantation von Organen oder Geweben, oder die Diagnose von zellulären Aktivierungszuständen durch den Nachweis von Veränderungen konsekutiver oder neu präsentierter integraler Membranproteine, von oberflächenaktiven Proteinen oder von Neoepitopen.

Werden Sender- und Empfängerpartikel, wie in Abb. 1B gezeigt, mit zwei verschiedenen Liganden beladen, beispielsweise der Sender mit Antikörpern gegen GP IIb und der Empfänger mit Antikörpern gegen GP IIIa, so wird nur dann ein Signal generiert, wenn sich die beiden unterschiedlichen Liganden in ausreichender Nähe zueinander befinden, d.h. im vorliegenden Beispiel, wenn der komplette Fibrinogen-Rezeptor GP IIb/IIIa auf der Zelle vorhanden ist.

Andere Komplexe, deren Nachweis von Interesse ist, können beispielsweise aus folgenden Komponenten bestehen: aus Gerinnungsenzymen und deren Cofaktoren, oder aus Gerinnungsenzymen und physiologisch aktiven Oberflächen, oder aus Komponenten des MHC (major histocompatibility complex), oder aus T3, Tr und T4 oder T8 auf Immunzellen, oder aus Komponenten des Komplementsystems, wie beispielsweise der MAC (membran attack complex), bestehend aus den Komplementfaktoren C5b, C6, C7, C8, C9 und Vitronection ("S Protein"). Im letzten Fall ist es beispielsweise denkbar, die aktuelle Lyseaktivität des Komplementsystems zu messen, indem Antikörper gegen C9 allein oder Kombinationen aus Antikörpern gegen zwei oder mehrere dieser Komponenten verwendet werden; beispielsweise kann dadurch die Vollständigkeit der Komplexausbildung (z.B. C5b-C6, C5b-C7, C5b-C8, C5b-C9) differenziert werden.

Das erfindungsgemäße Verfahren kann auch zur Vereinfachung des Nachweises von Mikroorganismen verwendet werden. Im folgenden soll das Prinzip vorliegender Erfindung am Beispiel des Chlamydiennachweises erläutert werden, wobei dem Fachmann unmittelbar klar ist, daß die Erfindung nicht auf den Nachweis dieses Mikroorganismus beschränkt ist.

Eine Vielzahl von Methoden zum Nachweis von Chlamydien wurden bereits beschrieben, wie etwa die Anzüchtung der Chlamydien in Zellkulturen, Immunoassays oder Nukleinsäure- (DNS-) Nachweisverfahren. Die immunologischen Testverfahren zielen beispielsweise auf den spezifischen Nachweis von Chlamydien für eine klinische Diagnosestellung. Zwei Methoden wurden hier im wesentlichen verwendet: eine erste, bei der enzymmarkierte Antikörper im zuvor freigesetzten Chlamydia-Antigen gemessen wurden; eine zweite, bei der an Objektträger fixierte Chlamydien, mit nicht freigesetztem noch an Chlamydien gebundenen Antigen mikroskopisch mittels fluoreszenzmarkierter Antikörper nachgewiesen wurden.

Für alle bisher bekannten Verfahren sind mehrere Schritte erforderlich, die bei der Probenvorbereitung für den Test oder im Test erfolgen. Einige sollen im folgenden genannt werden: In der Testvorbereitung müssen die nachzuweisenden Chlamydienantigene extrahiert werden. Dies geschieht mit Hilfe von Detergentien (siehe beispielsweise EP-0 392 865) oder mit Hilfe von Detergentien unter gleichzeitigen alkalischen Bedingungen (siehe beispielsweise EP-0 402 396).

Andere Verfahren benötigen, gegebenenfalls zusätzlich zur Extraktion, Waschschritte während des Testablaufs, um nicht gebundene chlamydiaspezifische Antikörper zu entfernen (siehe beispielsweise US-Patent Nr. 4 497 899).

Ein weiteres bisher bekanntes Verfahren erfordert eine Erhitzung auf 100 °C für 15 min, um chlamydiaspezifische Antigene für einen Nachweis freizusetzen (EP-0 371 049). Weitere andere Verfahren extrahieren Antigene, formen über spezifische Antikörper Immunkomplexe, die dann für den Nachweis ausfiltriert werden müssen, gefolgt von Waschschritten zur Entfernung ungebundener Antikörper (EP-0 451 687).

Ein weiteres bisher bekanntes Verfahren transportiert enzymatisch freigesetzte Antigene durch eine poröse Membran, in der chlamydiaspezifische Antikörper an die freigesetzten Antigene binden und durch nachfolgende Schritte nachgewiesen werden (EP-0 444 303).

Es wurde außerdem gefunden, daß durch Benutzung der LOCI-Technologie immunchemisch unlösliche Antigene direkt und ohne vorherige Freisetzung auf den Chlamydiazellen nachgewiesen werden können. Es reicht aus, Chlamydiazellen in einem Puffer zu dispergieren und in einem Aliquot dieser Zellsuspension Sender- und Empfängerpartikel immunchemisch zu binden und ohne weiteren Waschschritt das Signal zu messen. Da die beiden verschiedenen Antikörper unterschiedliche Strukturbestandteile in einer Chlamydienzelle erkennen, kann für einen erfindungsgemäßen Nachweis nur dann eine Signalbildung induziert werden, wenn beide - Sender- und Empfängerpartikel - nebeneinander an eine Chlamydienzelle gebunden werden können. Das bedeutet gleichzeitig, daß die Chlamydienzellstruktur nicht mehr zeitaufwendig destrukturiert und die gewünschten Antigene extrahiert werden müssen, damit die Einzelbestandteile der Chlamydienzellstruktur wie in anderen Testverfahren nachgewiesen werden können (siehe Beispiel 1). Dies erlaubt neben der Reduzierung von Arbeitsaufwand und Fehlerquellen auch die Applikation auf Routinegeräten, die auf Grund der Vorbehandlung bisher nicht möglich war.

Die vorliegende Erfindung betrifft daher auch ein Verfahren, demgemäß in einer Probe enthaltene Chlamydiayzellen mit Sender- und Empfängerpartikeln in Kontakt gebracht werden, wobei beide Partikelarten jeweils mindestens einen Liganden mit Bindungsaffinität für Chlamydiazellen und die Senderpartikel zusätzlich einen Sender sowie die Empfängerpartikel zusätzlich einen Empfänger tragen. Anschließend wird das Signal bestimmt, welches dann entsteht, wenn Sender und Empfänger in ausreichende Nähe zueinander gebracht werden.

Die nachfolgenden Beispiele sollen die vorliegende Erfindung noch weiter erläutern, jedoch in keiner Weise einschränken. Als Beispiel für ein homogenes, immunchemisches Verfahren wurde die LOCI-Technologie gewählt.

### Beispiel

Nachweis von Oberflächenantigenen von Chlamydien in Suspension:
Als Sender- und Empfänger wurden Sensitizer- bzw. Chemiluminescer-Partikel nach der LOCI-Technologie verwendet. Die Partikel wurden von der Syva Business Unit, Behring Diagnostics Inc., San Josè erworben. Die Herstellungsvorschrift folgte den in Patent EP 0 515 194 und in den Literaturstellen Ullman et al. Clin Chem. (1996) 42:9, 1518 - 1526 und Natl. Acad. Sci. (1994) 91, 5426 - 5430 beschriebenen Verfahren. Acceptor-Partikel wurden mit einem Lipopolysaccharid (LPS)-spezifischen Antikörper beschichtet. Das Beschichtungsverfahren ist bei Ullman et al. (1996) 42:9, 1518-1526 beschrieben. Parallel dazu wurde ein spezifischer Antikörper gegen das "Major outer membrane protein" (MOMP) mit Biotin markiert. Sensitizer-Partikel wurden mit Avidin beschichtet. Diese Verfahren sind ebenfalls bei Ullmann et al. (1996) 42:9, 1518 - 1526 beschrieben.
Vor dem Test wurden die Chlamydien, die Acceptorbeads, der biotinylierte Antikörper und die Sensitizer im folgenden Puffer verdünnt: 0,1 M Tris HCl; 0,5 M NaCl; 0,025 M EDTA; 1,6 % BSA (pH 7,6).
Zur Testdurchführung wurden die Komponenten wie folgt gemischt und inkubiert. Die dafür erforderliche instrumentelle Ausstattung ist in Ullman et al. (1996) 42:9, 1518 - 1526 beschrieben.
25 µl Chlamydiensuspension wurden mit 25 µl Acceptorbeads (100 µg/ml) gemischt und über 6 min bei 37 °C inkubiert. Danach wurden 35 µl biotinylierter Antikörper (10 µg/ml) zugegeben und erneut für 6 min bei 37 °C inkubiert. Anschließend wurden 50 µl Sensitizer-Partikel (400 µg/ml) zugegeben und die emittierte Chemolumineszenz in einem Luminometer bestimmt.
Zum Vergleich wurde ein Testansatz ohne Chlamydien verwandt. Die Signale der beiden Ansätze waren:

| | |
|---|---|
| Signal mit Probenpuffer (Kontrolle): | 8698 |
| Signal mit Chlamydiazellen: | 13221 |

## Patentansprüche

1. Homogenes immunchemisches Verfahren, welches nicht auf dem Prinzip der Durchflußzytometrie beruht und welches auf anderen als auf dem Prinzip der Durchflußzytometrie beruhenden Geräten ausgewertet wird, zum gleichzeitigen Nachweis oder zur gleichzeitigen Bestimmung voneinander verschiedener festphasenassoziierter Faktoren, nämlich mindestens eines ersten festphasenassoziierten Faktors Fx und eines zweiten festphasenassoziierten Faktors Fy, wobei Fx und Fy mit derselben Festphase assoziiert sind, in einer Probe, **dadurch gekennzeichnet, daß** die Probe mit einem ersten stabilen Komplex, bestehend aus mindestens einem Liganden Lx, der Bindungsaffmität für Fx besitzt und einem Sender S, wobei Lx zusammen mit S an einem ersten Partikel immobilisiert ist sowie einem zweiten stabilen Komplex, bestehend aus mindestens einem Liganden Ly, der Bindungsaffinität für Fy besitzt und einem Empfänger E, wobei Ly zusammen mit E an einem zweiten Partikel immobilisiert ist, in Kontakt gebracht wird, so daß sich Komplexe Fx-Lx-S und Py-Ly-E ausbilden, und daß das Signal bestimmt wird, welches dann entsteht, wenn sich S und E in ausreichender Nähe zueinander befinden und worin die Festphase eine in einer Flüssigkeit suspendierte Zelle und Fx und/oder Fy ein integrales Membranprotein, ein membranassoziiertes Protein oder ein Lipid ist.

2. Verfahren gemäß Anspruch 1, worin unter Zelle eine Blutzelle, wie ein Erythrozyt, Leukozyt, Granulozyt, Lymphozyt, Monozyt, Thrombozyt, oder eine Zelle aus einem anderen Gewebe oder Organ verstanden wird.

3. Verfahren gemäß Anspruch 1, worin unter Zelle eine körperfremde Zelle verstanden wird.

4. Verfahren gemäß Anspruch 1, worin unter Zelle Bakterium, Parasit oder Virus verstanden wird.

5. Verfahren nach Anspruch 1, worin das integrale Membranprotein ein Integrin oder Selektin ist, oder aus dem MHC-Komplex stammt, oder ein anderes Protein gemäß der "cluster designation" ist.

6. Verfahren nach Anspruch 1, worin das membranassoziierte Protein Fibrinogen, ein Antikörper, ein Komplementfaktor, ein Lektin, ein prozessiertes Antigen im MHC-Komplex, ein Enzym aus dem Gerinnungssystem oder ein Protein aus der Familie der Annexine ist.

7. Verfahren nach Anspruch 1, worin das Lipid aus der Gruppe der Acylglycerine, Phosphoglyceride, Sphingolipide, Wachse, Terpene, Steroide und/oder Prostaglandine stammt.

8. Verfahren nach Anspruch 1, worin das Lipid ein Phospholipid und ein Ligand ein Protein aus der Familie der Annexine oder ein affines Protein des Gerinnungssystems ist.

9. Verfahren nach Anspruch 8, worin das Phospholipid Phosphatidylserin oder Phosphatidylethanolamin und L Protein C oder Protein S ist.

10. Verfahren nach Anspruch 1, worin ein Ligand über einen vermittelnden Bindungspartner an Fx oder Fy bindet.

11. Verfahren nach Anspruch 1, **dadurch gekennzeichnet, daß** Lx oder Ly über eine Biotin-Avidin-Brücke an Partikel gebunden wird.

12. Verfahren nach Anspruch 1, worin Lx oder Ly ein Antikörper, Antigen, Lektin, Coenzym, Apoprotein, Ligand eines Rezeptors, Substratanaloges oder Annexin sein kann.

13. Verfahren nach Anspruch 1, **dadurch gekennzeichnet, daß** zwischen S und E ein Energietransfer stattfindet.

14. Verfahren nach Anspruch 13, **dadurch gekennzeichnet, daß** der Energietransfer durch radioaktive Prozesse, durch Anregung photosensibler Farbstoffe und **dadurch** bedingter direkter oder indirekter Blektronenübertragung erfolgen kann.

15. Verfahren nach Anspruch 14, **dadurch gekennzeichnet, daß** die Elektronenübertragung mittels aktivierten Sauerstoffs erfolgt.

16. Verfahren nach Anspruch 13, **dadurch gekennzeichnet, daß** der Energietransfer beim Empfänger eine Lumineszenz, Chemilumineszenz oder Fluoreszenz erzeugt.

17. Verfahren nach Anspruch 1, **dadurch gekennzeichnet, daß** eine die Signalentstehung modulierende Substanz zugesetzt wird.

18. Verfahren nach Anspruch 1, **dadurch gekennzeichnet, daß** es der Charakterisierung von Zelltypen, Untergruppen oder Aktivierungszuständen von Zellen dient.

19. Verfahren nach Anspruch 1, **dadurch gekennzeichnet, daß** der Nachweis von Oberflächenantigenen auf Bakterien der Identifikationen von Krankheitserregern dient.

20. Verfahren nach Anspruch 1, **dadurch gekennzeichnet, daß** es dem Nachweis von Oberflächenmarkern auf Zellen dient.

21. Verfahren nach Anspruch 19, **dadurch gekennzeichnet, daß** es dem Nachweis von Chlamydien dient.

22. Verfahren nach Anspruch 1, **dadurch gekennzeichnet, daß** es zur Typisierung von Geweben oder der Charakterisierung der Gewebekompatibilität dient.

## Claims

1. A homogeneous immunochemical process which does not rely on the principle of flow cytometry and which is evaluated on instruments other than those relying on the principle of flow cytometry, for the simultaneous detection or for the simultaneous determination of mutually different solid-phase-associated factors, namely at least one first solid-phase-associated factor Fx and at least one second solid-phase-associated factor Fy, where Fx and Fy are associated with the same solid phase, in a sample, **characterized in that** it comprises bringing the sample into contact with a first stable complex consisting of at least one ligand Lx, which has binding affinity for Fx, and a transmitter T, Lx being immobilized together with T on a first particle, and also a second stable complex consisting of at least one ligand Ly, which has binding affinity for Fy, and a receiver R, Ly being immobilized together with R on a second particle, to form complexes Fx-Lx-T and Fy-Ly-R, and determining the signal which results when T and R are sufficiently close to one another, and wherein the solid phase is a cell suspended in a liquid and Fx and/or Fy is an integral membrane protein, a membrane-associated protein or a lipid.

2. The process as claimed in claim 1, wherein cell is understood as meaning a blood cell, such as an erythrocyte, leucocyte, granulocyte, lymphocyte, monocyte, thrombocyte or a cell from another tissue or organ.

3. The process as claimed in claim 1, wherein cell is understood as meaning an exogenous cell.

4. The process as claimed in claim 1, wherein cell is understood as meaning bacterium, parasite or virus.

5. The process as claimed in claim 1, wherein the integral membrane protein is an integrin or selectin, or derives from the MHC complex, or is another protein according to the cluster designation.

6. The process as claimed in claim 1, wherein the membrane-associated protein is fibrinogen, an antibody, a complement factor, a lectin, a processed antigen in the MHC complex, an enzyme of the clotting system or a protein from the annexins family.

7. The process as claimed in claim 1, wherein the lipid derives from the acylglycerols, phosphoglycerides, sphingolipids, waxes, terpenes, steroids and/or prostaglandins group.

8. The process as claimed in claim 1, wherein the lipid is a phospholipid and a ligand is a protein from the annexins family or a reactive protein of the clotting system.

9. The process as claimed in claim 8, wherein the phospholipid is phosphatidylserine or phosphatidylethanolamine and L is protein C or protein S.

10. The process as claimed in claim 1, wherein the ligand binds to Fx or Fy via a mediatory binding component.

11. The process as claimed in claim 1, **characterized in that** Lx or Ly is bound to particles via a biotin-avidin bridge.

12. The process as claimed in claim 1, wherein Lx or Ly can be an antibody, antigen, lectin, coenzyme, apoprotein, ligand of a receptor, substrate analog or annexin.

13. The process as claimed in claim 1, **characterized in that** an energy transfer takes place between S and E.

14. The process as claimed in claim 13, **characterized in that** the energy transfer can be effected by radioactive processes, or by excitation of photosensitive dyes and direct or indirect electron transfer caused thereby.

15. The process as claimed in claim 14, **characterized in that** the electron transfer is effected by means of activated oxygen.

16. The process as claimed in claim 13, **characterized in that** the energy transfer produces luminescence, chemiluminescence or fluorescence in the receiver.

17. The process as claimed in claim 1, **characterized in that** a substance modulating signal formation is added.

18. The process as claimed in claim 1, **characterized in that** it is used for the characterization of cell types, subgroups or activation states of cells.

19. The process as claimed in claim 1, **characterized in that** the detection of surface antigens on bacteria is used for the identification of pathogens.

20. The process as claimed in claim 1, **characterized in that** it is used for the detection of surface markers on cells.

21. The process as claimed in claim 19, **characterized in that** it is used for the detection of Chlamydia.

22. The process as claimed in claim 1, **characterized in that** it is used for the typing of tissues or tissue compatibility characterization.

## Revendications

1. Procédé iminunochimique homogène, qui ne repose pas sur le principe de la, cytométrie de flux et qui est mis en oeuvre sur des appareils autres que ceux reposant sur le principe de la cytoniétrie de flux, pour la détection simultanée et la détermination simultanée dans un échantillon de facteurs associés à une phase solide différents l'un de l'autre, à savoir au moins un premier facteur associé à une phase solide Fx et un deuxième facteur associé à une phase solide Fy, Fx et Fy étant associés à la même phase solide, **caractérisé en ce que** l'échantillon est mis en contact avec un premier complexe stable consistant en au moins un ligand Lx ayant une affinité de liaison pour Fx et un émetteur S, Lx étant immobilisé avec S sur une première particule, ainsi qu'avec un deuxième complexe stable consistant en au moins un ligand Ly ayant une affinité de liaison pour Fy et un récepteur E, Ly étant immobilisé avec E sur une deuxième particule, de sorte qu'on obtient les complexes Fx-Lx-S et Fy-Ly-E, et **en ce qu'**on détermine le signal qui est obtenu lorsque S et E se trouvent à proximité suffisante l'un de l'autre, la phase solide étant une cellule en suspension dans un liquide et Fx et/ou Fy étant une protéine membranaire intégrale, une protéine associée à une membrane ou un lipide.

2. Procédé selon la revendication 1, dans le lequel on entend par cellule une cellule sanguine, telles qu'un érythrocyte, un leucocyte, un granulocyte, un lymphocyte, un monocyte, un thrombocyte ou une cellule provenant d'un autre tissu ou organe.

3. Procédé selon la revendication 1, dans lequel on entend par cellule une cellule exogène.

4. Procédé selon la revendication 1, dans lequel on entend par cellule une bactérie, un parasite ou un virus.

5. Procédé selon la revendication 1, dans lequel la protéine membranaire intégrale est une intégrine ou une sélectine, ou provient du complexe MHC, ou est une autre protéine selon la "cluster designation".

6. Procédé selon la revendication 1, dans lequel la protéine associée à une membrane est un fibrirogène, un anticorps, un facteur de complément, une lectine, une antigène traité dans le complexe MHC, un enzyme provenant du système de coagulation ou une protéine de la famille des annexines.

7. Procédé selon la revendication 1, dans lequel le lipide provient du groupe des acyl-glycérols, phosphoglycérides, sphingolipides, cires, terpènes, stéroïdes et/ou prostaglandines.

8. Procédé selon la revendication 1, **caractérisé en ce que** le lipide est un phospholipide et un ligand est une protéine appartenant à la famille des annexines, ou une protéine affine du système de coagulation.

9. Procédé selon la revendication 8, dans lequel le phospholipide est une phosphatidylsérine ou une phosphatidyléthanolamine et est une protéine C ou une protéine S.

10. Procédé selon la revendication 1, dans lequel un ligand se lie à Fx ou Fy par l'intermédiaire d'un ligand médiateur.

11. Procédé selon la revendication 1, **caractérisé en ce que** Lx ou Ly sont liés aux particules par l'intermédiaire d'un pont biotine-avidine.

12. Procédé selon la revendication 1, dans lequel Lx ou Ly peut être un anticorps, un antigène, une lectine, un coenzyme, une apoprotéine, le ligand d'un récepteur, un analogue de substrat ou une annexine.

13. Procédé selon la revendication 1, **caractérisé en ce qu'**un transfert d'énergie se produit entre S et E.

14. Procédé selon la revendication 13, **caractérisé en ce que** le transfert d'énergie peut se faire par des processus radioactifs, par excitation de colorants photosensibles, entraînant directement ou indirectement un transfert d'électrons.

15. Procédé selon la revendication 14, **caractérisé en ce que** le transfert d'électrons se fait au moyen d'oxygène activé.

16. Procédé selon la revendication 13, **caractérisé en ce que** le transfert d'énergie produit chez le récepteur une luminescence, une chimiolmninescence ou une fluorescence.

17. Procédé selon la revendication 1, **caractérisé en ce qu'**on utilise une substance modulant la formation du signal.

18. Procédé selon la revendication 1, **caractérisé en ce qu'**il sert à la caractérisation de types de cellules, de sous-groupes ou d'états d'activation de cellules.

19. Procédé selon la revendication 1, **caractérisé en ce que** la détection d'antigènes de surface sur des bactéries sert à l'identification d'agents pathogènes.

20. Procédé selon la revendication 1, **caractérisé en ce qu'**il sert à la détection de marqueurs de surface sur des cellules;

21. Procédé selon la revendication 19, **caractérisé en ce qu'**il sert à la détection des chlamydia.

22. Procédé selon la revendication 1, **caractérisé en ce qu'**il sert au typage des tissus ou à la caractérisation de la compatibilité des tissus.
